# EUROPEAN PATENT APPLICATION

(11) **EP 4 035 736 A1**
(43) Date of publication of application: **03.08.2022**
(21) Application number: 20867802.9
(22) Date of filing: 26.06.2020
(51) Int. Cl.: A61P 43/00, A23L 33/21, C12N 1/20, A61K 31/734

(54) **PREBIOTIC COMPOSITION FOR BUTYRIC ACID BACTERIA**

(30) Priority: 27.09.2019 JP 2019176534
(71) Applicant: Morinaga Milk Industry Co., Ltd., Minato-ku Tokyo 108-8384 (JP)
(72) Inventor: ODAMAKI, Toshitaka, Zama-shi, Kanagawa 252-8583 (JP); MURAKAMI, Ryuta, Zama-shi, Kanagawa 252-8583 (JP); HASHIKURA, Nanami, Zama-shi, Kanagawa 252-8583 (JP)
(74) Representative: Hoffmann Eitle
(86) International application number: PCT/JP2020/025251
(87) International publication number: WO 2021/059639

(57) **Abstract**

An aspect is to provide a prebiotic for growing a butyrate-producing bacterium. A prebiotic composition for a butyrate-producing bacterium includes alginic acid and/or a salt thereof having an average molecular weight of 10000 or less.

## Description

### Technical Field

The present invention relates to a prebiotic composition for a butyrate-producing bacterium.

### Background Art

In recent years, the relevance of enteric bacteria to overall health has been attracting attention and has been studied worldwide. The term "probiotics", which is a term related to improvement of the enteric environment, generally refers to living microorganisms which improve the balance of the enteric flora and which thus achieve useful effects in humans, and bifidobacteria and the like are generally known. "Prebiotics", which are assimilated by the probiotics, generally are not decomposed or absorbed in the upper gastrointestinal tract and are selective nutrient sources for useful commensal bacteria in the large intestine, promote the growth thereof, improve and maintain the healthy balance of the enteric flora composition in the large intestine, and serve to improve and maintain human health.

One of the useful commensal bacteria in the large intestine is a butyrate-producing bacterium. Butyric acid is a short-chain fatty acid present in the intestines and serves as the main nutrient providing energy to the large intestinal cells. Butyric acid also serves as a cell mediator which adjusts various functions not only in the intestines, such as gene expression of the host, cell differentiation, development of intestinal tissues, immunomodulation, reduction in oxidative stress and diarrhea control (NPL 1).

It is useful to ingest butyric acid forhealth. However, because butyric acid emits very strong unpleasant odor, its intake in the form of a food, a pharmaceutical product, or the like is not practical. Moreover, the major butyrate-producing bacteria living in the intestines are extremely oxygen sensitive, and thus *in vitro* cultivation thereof is extremely difficult. Therefore, it is also difficult to formulate a butyrate-producing bacterium for ingestion (NPL 2) .

### Citation List

### Non Patent Literature

NPL 1: Cell. 2016 Jun 2;165(6):1332-1345
NPL 2: Best. Pract. Res. Clin. Gastroenterol. 2017 Dec;31(6):643-648.

### Summary of Invention

### Technical Problem

Considering the circumstances, the present inventors have thought that promoting the growth of butyrate-producing bacteria living in the intestines would be useful for maintaining and improving overall health. Accordingly, an aspect of the invention is to provide a prebiotic that promotes the growth of *Faecalibacterium prausnitzii,* which is a butyrate-producing bacterium.

### Solution to Problem

As a result of intensive research to solve the problem, the present inventors have found that alginic acid and/or a salt thereof can promote the growth of butyrate-producing bacteria including *Faecalibacterium prausnitzii* remarkably in the intestines. Moreover, the inventors have also made findings that *Faecalibacterium prausnitzii* cannot assimilate alginic acid and/or a salt thereof above a certain size and does not grow in an environment other than in the intestines, namely in the absence of bacteria other than butyrate-producing bacteria. Based on the findings, the inventors have reached the idea that alginic acid and/or a salt thereof having a particular size is effective as a prebiotic for *Faecalibacterium prausnitzii.* The invention has been thus completed.

That is, a first embodiment of the invention is a prebiotic composition for *Faecalibacterium prausnitzii* comprising alginic acid and/or a salt thereof having an average molecular weight of 10000 or less.

In the embodiment, the butyrate-producing bacterium is preferably a bacterium of the genus *Faecalibacterium.*

The composition of the embodiment preferably comprises the alginic acid and/or the salt thereof in an amount of 0.1 mass% or more of the entire composition.

The composition of the embodiment is preferably a food or a drink.

The composition of the embodiment is preferably a pharmaceutical product.

A second embodiment of the invention is a method for producing a prebiotic composition for a butyrate-producing bacterium comprising a step of hydrolyzing long-chain alginic acid and/or a salt thereof to reduce the weight average molecular weight to 10000 or less.

In the embodiment, the butyrate-producing bacterium is preferably a bacterium of *Faecalibacterium.*

A third embodiment of the invention is a method for producing a prebiotic food or drink for a butyrate-producing bacterium comprising a step of hydrolyzing long-chain alginic acid and/or a salt thereof to reduce the weight average molecular weight to 10000 or less and a step of adding the alginic acid and/or the salt thereof having an average molecular weight of 10000 or less obtained in the step to a food or drink material.

In the embodiment, the butyrate-producing bacterium is preferably a bacterium of *Faecalibacterium.*

### Advantageous Effects of Invention

According to the invention, a prebiotic which can efficiently promote the growth of *Faecalibacterium prausnitzii* is provided. Butyric acid which is produced by *Faecalibacterium prausnitzii* functions as a cell mediator and maintains and improves overall health. Thus, the invention is industrially extremely useful.

### Brief Description of Drawings

[Fig. 1] HPLC charts comparing the molecular weights of sodium alginate (sample 1) and an external standard product (pullulan).
[Fig. 2] A graph showing the changes in turbidities over time of the media obtained by a monoculture of *Faecalibacterium prausnitzii* to which sodium alginates having different molecular weights (sample 1 (black circle) and sample 2 (white circle)) and a guar gum hydrolysate (sample 3 (white triangle)) were added or in a medium to which no sample was added (white diamond).
[Fig. 3] HPLC charts of the medium obtained after 0 or 48 hours of cultivation of monoculture of *Faecalibacterium prausnitzii* in a medium to which sodium alginate (sample 1) was added and pullulan standards.
[Fig. 4] HPLC charts of the medium obtained after 0 or 48 hours of cultivation of monoculture of *Faecalibacterium prausnitzii* in a medium to which sodium alginate (sample 1) was added and sample 1.

### Description of Embodiments

Next, the invention is explained in detail. However, the invention is not limited to the following embodiments and can be changed freely within the scope of the invention.

The composition of the invention includes alginic acid and/or a salt thereof (also referred to as "alginic acid compounds" below) having an average molecular weight of 10000 or less. The upper limit of the weight average molecular weight is preferably 9000 or less, more preferably 8000 or less, further preferably 7000 or less, particularly preferably 5000 or less. The lower limit of the weight average molecular weight is not particularly limited but is generally 194 or more. Here, the weight average molecular weight of an alginate is a value in terms of alginic acid.

The composition of the invention may include alginic acid and/or a salt thereof having a higher average molecular weight than the value range as long as the effects are not impaired.

In the present specification, the average molecular weight of an alginic acid compound is a value measured by the HPLC method conducted under the following conditions.
Measurement Apparatus: Ultimate 3000 (manufactured by Thermo Inc.)
Detection: Refracto Max 521 detector (manufactured by Thermo Inc.),
Column: TSKgel G3000PW (manufactured by Tosoh Corporation)
Mobile Phase: aqueous 0.1M sodium nitrate solution
Flow Rate: 0.3 mL/min
Column Temperature: 40°C
Standard: STANDARD P-82 (manufactured by Shodex)

The molecular weight measured by HPLC conducted under the above conditions is an "apparent" value which may be different from the "true" molecular weight calculated from the actual degree of polymerization.

As shown in the Examples described below, the molecular weight of an alginate oligosaccharide having a degree of polymerization of 4 to 5 measured under the above conditions is about 3500.

Therefore, the alginic acid compounds included in the composition of the invention may also be oligosaccharides having a degree of polymerization of preferably 20 or less, more preferably 10 or less, further preferably 8 or less, particularly preferably 5 or less.

The alginic acid compounds included in the composition of the invention may have a molecular weight measured by the HPLC method conducted under the following conditions of less than 40000. The upper limit thereof is preferably 35000 or less, more preferably 30000 or less, further preferably 25000 or less, particularly preferably 22000 or less. The lower limit of the molecular weight measured by the HPLC method conducted under the following conditions is not particularly limited but is generally 194 or more, more preferably 5000 or more, further preferably 10000 or more. Here, the molecular weight of an alginate is a value in terms of alginic acid.

The conditions here are as follows.
Measurement Apparatus: Ultimate 3000 (manufactured by Thermo Inc.)
Detection: Corona Charged Aerosol Detector (manufactured by Thermo Inc.),
Column: TSKgel G6000PW (manufactured by Tosoh Corporation)
Mobile Phase: aqueous 0.1% ammonium acetate solution
Flow Rate: 0.4 mL/min
Column Temperature: 40°C
Standard: STANDARD P-82 (manufactured by Shodex)

The molecular weight measured by HPLC conducted under the conditions is also an "apparent" value which may be different from the "true" molecular weight calculated from the actual degree of polymerization.

Alginic acid is a polysaccharide present in marine algae, such as tangle weed and *Undaria pinnatifida,* and is widely used, including in foods as a thickening and stabilizing agent. Alginic acid has a linear structure composed of β-D-mannuronic acid and α-L-guluronic acid linked by 1-4 bonds. The degree of polymerization of alginic acid varies with its origin.

The salt of alginic acid is the sodium salt, the potassium salt, the calcium salt, the ammonium salt or the like and is not particularly limited in the invention, but the sodium salt and the potassium salt are preferable in view of the solubility in water.

In general, alginic acid compounds can be obtained by extraction from a marine alga or the like, and those commercially available can also be obtained. These compounds generally have a high molecular weight of about 40000 to multimillion. According to the experiment of the present inventors, it was confirmed that *Faecalibacterium prausnitzii* alone cannot assimilate the "long-chain alginic acid compounds" having average molecular weights exceeding 10000.

An alginic acid compound having a average molecular weight of 10000 or less which is used as an active ingredient of the prebiotic composition of the invention can be obtained by hydrolyzing "a long-chain alginic acid compound". That is, a method for producing a prebiotic composition for *Faecalibacterium prausnitzii* including a step of hydrolyzing long-chain alginic acid and/or a salt thereof to reduce the average molecular weight to 10000 or less, which is another embodiment of the invention, is provided.

The hydrolysis method is acid hydrolysis, a method by a hydrolase, a method by a bacterium having a hydrolase, or the like.

Specifically, acid hydrolysis is conducted by adding a weak acid such as acetic acid to an aqueous solution of a long-chain alginic acid compound and heating to a high temperature, such as 100°C or higher, for several hours but is not limited to this method.

Examples of the hydrolase include commercial alginate lyase and the like, and a long-chain alginic acid compound is hydrolyzed by reacting such a hydrolase.

Examples of the bacterium having a hydrolase include commercial *Flavobacterium* bacteria and the like, and hydrolysis is conducted by culturing such a bacterium under appropriate conditions in the presence of a long-chain alginic acid compound.

The amount of alginic acid compounds having an average molecular weight of 10000 or less in the composition of the invention may be appropriately set based on the form of the composition and is not particularly limited, but for example, the amount is preferably 0.1 mass% or more of the entire composition, more preferably 1 mass% or more. The upper limit of the amount of alginic acid compounds having an average molecular weight of 10000 or less is not particularly restricted but may be, for example, 5 mass% or less or 100 mass% or less of the entire composition. In the case of an alginate, these values are in terms of alginic acid. These amounts include the values during production of the composition of the invention, the values during distribution and the values for intake (administration).

The composition of the invention is used as a prebiotic for a butyrate-producing bacterium. That is, the composition is assimilated by a butyrate-producing bacterium and can promote the growth thereof.

Butyrate-producing bacterium is a generic term for bacteria which produce butyric acid. The butyrate-producing bacterium in the invention is not particularly limited. Examples include bacteria belonging to the genus *Faecalibacterium,* and more specific examples include *Faecalibacterium prausnitzii,* which is present in the human intestines, and the like.

The composition of the invention can be useful for a subject having a disease or a pathological condition which can be prevented or improved through an increase in butyric acid in the body, or a disease or a pathological condition which is caused by a decrease in butyric acid in the body. For example, the composition can be for intestinal regulation, for immunomodulation, for reduction in oxidative stress, for prevention/improvement of diarrhea, for inflammatory bowel disease, for prevention of large intestine cancer or the like.

Another embodiment of the invention is use of alginic acid and/or a salt thereof having an average molecular weight of 10000 or less in the manufacture of a prebiotic composition for a butyrate-producing bacterium.

Another embodiment of the invention is use of alginic acid and/or a salt thereof having an average molecular weight of 10000 or less in promoting the growth of *Faecalibacterium prausnitzii.*

Another embodiment of the invention is alginic acid and/or a salt thereof having an average molecular weight of 10000 or less for use in promoting the growth of *Faecalibacterium prausnitzii.*

A method for growing *Faecalibacterium prausnitzii* includes administering alginic acid and/or a salt thereof having a average molecular weight of 10000 or less to an animal. The animal is not particularly limited but is generally a human.

The timing of intake (administration) of the composition of the invention is not particularly limited and can be appropriately selected depending on the condition of the subject of the administration.

The dosage of the composition of the invention is appropriately selected based on the age of the subject of the intake (administration), the gender, the condition, other conditions, and the like. A standard amount of alginic acid compounds having an average molecular weight of 10000 or less is an amount falling in the range of preferably 1 to 300 mg/kg/day, more preferably 20 to 50 mg/kg/day.

Regardless of the amount or the period of intake (administration), the medicine can be administered once a day or in multiple divided portions.

The route of intake (administration) of the composition of the invention may be an oral or parenteral route but is generally an oral route. The parenteral intake (administration) is rectal administration or the like.

In an embodiment, the composition of the invention may include a butyrate-producing bacterium with the alginic acid compounds having an average molecular weight of 10000 or less. The composition of the invention may be taken in combination with a butyrate-producing bacterium or with a preparation including a butyrate-producing bacterium. As a result of the embodiment, an effect of promoting the growth of the butyrate-producing bacterium in an intestine and an effect of thus increasing butyric acid are expected.

In these embodiments, the butyrate-producing bacterium is preferably a living bacterium.

When the composition of the invention is an orally taken composition, the composition is preferably a food or a drink in an embodiment.

The form and the property of the food or the drink are not particularly restricted as long as the food or the drink does not impair the effects of the invention and can be orally taken, and the food or the drink can be produced by a general method using a material which is generally used for producing a food or a drink except that the alginic acid compounds having an average molecular weight of 10000 or less are added.

Because a generally available alginic acid compound has a long chain as described above, a prebiotic food or drink for a butyrate-producing bacterium can also be produced by a method including a step of hydrolyzing long-chain alginic acid and/or a salt thereof to reduce the average molecular weight to 10000 or less, and a step of adding the alginic acid and/or the salt thereof having an average molecular weight of 10000 or less obtained in the step to a food or drink material, which is another embodiment of the invention.

The food or the drink is not limited regarding the form such as liquid, paste, gel solid, or powder. Examples include the following examples: tablet candies; liquid foods (nutrition products for tube feeding); wheat products such as breads, macaroni, spaghetti, noodles, cake mixes, frying flours, and bread crumbs; instant foods such as instant noodles, cup noodles, retort-pouched/prepared foods, prepared canned foods, microwave foods, instant soups/stews, instant miso soups/clear Japanese soups, canned soups, freeze-dried foods, and other instant foods; processed agricultural products such as canned agricultural products, canned fruits, jams/marmalades, pickles, cooked beans, dried agricultural products, and cereals (processed grains); processed fishery products such as canned fishery products, fish hams/sausages, fishery paste products, fishery delicacies, and *Tsukudani* (foods boiled down in sweetened soy sauce); processed livestock products such as canned livestock products/pastes and livestock hams/sausages; milk/dairy products such as processed milk, milk beverages, yogurts, lactic acid bacteria beverages, cheeses, ice creams, modified milk powders, creams, and other dairy products; oils and fats such as butter, margarine, and vegetable oils; basic condiments such as soy sauce, soybean paste, sauces, processed tomato condiments, *Mirin* (sweet sake for seasoning), and vinegars; compound flavor enhancers/foods such as cooking mixes, curry roux, sauces, dressings, noodle broths, spices, and other compound flavor enhancers; frozen foods such as frozen food materials, semi-cooked frozen foods and cooked frozen foods; confectioneries such as caramels, candies, chewing gums, chocolates, cookies, biscuits, cakes, pies, snacks, crackers, Japanese-style confectioneries, rice confectioneries, bean confectioneries, desserts, jellies, and other confectioneries; luxury beverages such as carbonated drinks, natural juices, fruit juices, fruit juice-comprising soft drinks, fruit flesh drinks, fruit granule-comprising fruit juices, vegetable drinks, soy milk, soy milk drinks, coffee drinks, tea drinks, drink powders, concentrated drinks, sport drinks, nutritional drinks, alcohols, and other luxury beverages, other commercial foods such as baby foods, *Furikake* (dry Japanese seasonings) and seasonings for *Chazuke* (boiled rice with hot tea); infant modified milk powder; enteral nutrition products; functional foods (foods for specified health uses and foods with nutrient function claims); and the like.

An embodiment of the food or the drink can be feed. The feed is pet food, livestock feed, fish farming feed, or the like.

The form of the feed is not particularly restricted and includes, in addition to the alginic acid compounds having an average molecular weight of 10000 or less, for example: an alginic acid compound having an average molecular weight of more than 194, grain such as corn, wheat, barley, rye, and milo; vegetable oil cake such as soybean oil cake, rapeseed oil cake, coconut oil cake, and linseed oil cake; bran such as oat bran, wheat bran, rice bran, and defatted rice bran; a food manufacturer's by-product such as corn gluten meal and corn jam meal; animal feed such as fish powder, defatted milk powder, whey, yellow grease, and tallow; yeast such as torula yeast and brewer's yeast; mineral feed such as tertiary calcium phosphate and calcium carbonate; an oil or a fat; a single amino acid; a saccharide; or the like.

When the composition of the invention is an embodiment of a food or a drink (including feed), the composition can be provided/sold as a food or a drink labeled with use as a prebiotic for a butyrate-producing bacterium or for growing a butyrate-producing bacterium in an intestine.

The "labeling" act includes all acts hat inform a consumer of the use, and all the expressions which remind of/cause one to guess the use are also "labeling" acts of the invention, regardless of the purposes of labeling, the contents of labeling, the objects to be labeled, the media, and the like.

The "label" preferably contains an expression which allows a consumer to directly recognize the use. Specific examples include an act of transferring an article in which the use is described on a product regarding the food or the drink or packaging of a product, delivering such an article, displaying such an article for transfer or delivery, or importing such an article, an act of displaying or distributing an advertisement of a product, a price list or a business document with a description of the use thereon, or providing information with such contents with a description of the use by an electromagnetic method (internet or the like), and another act.

The content of the label is preferably approved by the administration or the like (for example, a label approved based on a system provided by the administration and provided in the form based on the approval or the like). It is preferable to label with such a content on packaging, a container, a catalogue, a brochure, an advertisement material in a sales site such as POP, other documents, or the like.

The "labels" also include labels with health foods, functional foods, enteral nutrition products, food for special dietary uses, food with health claims, foods for specified health uses, foods with nutrient function claims, foods with function claims, quasi-drugs, and the like. In particular, the labels are labels approved by the Consumer Affairs Agency, such as labels approved by the systems for foods for specified health uses, foods with nutrient function claims or foods with function claims and labels approved by a similar system, and the like. Specific examples include a label with foods for specified health uses, a label with qualified foods for specified health uses, a label indicating influence on the structure or the function of a body, a label with reduction of disease risk, a label with a scientifically grounded function and the like. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The labels are, for example, labels with "for those who wish to increase butyrate-producing bacteria in the stomach", "for those who wish to increase bacteria of *Faecalibacterium",* "intestinal regulation effect with butyric acid", and the like.

In an embodiment, the composition of the invention can be a pharmaceutical product.

The administration route of the pharmaceutical product may be an oral or parenteral route but is preferably an oral route. The parenteral intake (administration) is rectal administration or the like.

Regarding the form of the pharmaceutical product, the composition can be appropriately formulated into a desired dosage form depending on the administration method. For example, in the case of oral administration, the composition can be formulated into a solid preparation such as powder, granules, tablets and capsules, a liquid preparation such as a solution, a syrup, a suspension, and an emulsion, or the like. In the case of parenteral administration, the composition can be formulated into a suppository, ointment, an injection, or the like.

For the formulation, in addition to the alginic acid compounds having an average molecular weight of 10000 or less, a component which is generally used for formulation such as excipients, pH-adjusting agents, colorants, and corrigents can be used. Another medicinal component, a prebiotic which is known or will be found in the future or the like can also be used in combination.

In addition, the formulation can be appropriately conducted by a known method depending on the dosage form. For the formulation, a carrier for formulation can be appropriately blended and formulated.

Examples of the excipients include: saccharide derivatives such as lactose, sucrose, glucose, mannitol,, and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose, and carboxymethyl cellulose calcium; gum arabic; dextran; pullulan; silicate derivatives such as light silicic anhydride, synthetic aluminum silicate, and magnesium aluminometasilicate; phosphate derivatives such as calcium phosphate; carbonate derivatives such as calcium carbonate; sulfate derivatives such as calcium sulfate; and the like.

Examples of binders include, in addition to the excipients: gelatin; polyvinylpyrrolidone; macrogol; and the like.

Examples of disintegrating agents include, in addition to the excipients, chemically modified starch or cellulose derivatives such as croscarmellose sodium, sodium carboxymethyl starch and cross-linked polyvinylpyrrolidone and the like.

Examples of lubricants include: talc; stearic acid; metal stearates such as calcium stearate and magnesium stearate; colloidal silica; waxes such as peegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of flavoring agents include sweeteners, acidulants, aromas, and the like.

In this regard, the carriers used in the case of a liquid preparation for oral administration include solvents such as water and the like.

The timing for taking the pharmaceutical product of the invention is not particularly limited, and examples include before a meal, after a meal, between meals, before bedtime, and the like.

### Examples

The invention is explained more specifically below using Examples, but the invention is not limited to these Examples.

### <Production Example>

### (1) Acquisition of Long-Chain Sodium Alginate Hydrolysate

One gram of a commercial sodium alginate material ("ULV-L3 (molecular weight of 40000 to 60000)" (manufactured by KIMICA Corporation)) was dissolved in 99.7 mL of MilliQ water. Then, 0.3 mL of acetic acid (Kokusan Chemical Co., Ltd.) was added, and the mixture was autoclaved at 104°C for 7.5 hours. Next, after centrifugation at 8000 × g for five minutes, the supernatant was collected. The supernatant was subjected to SpeedVac to remove all the acetic acid and 70% of the water content, and an aqueous solution of a long-chain sodium alginate hydrolysate (sample 1) was thus obtained.

### (2) Estimation of Molecular Weight of Sodium Alginate

The molecular weight of the sodium alginate (sample 1) obtained in (1) was determined by HPLC. Sample 1 was filtered through a 0.22 µm filter (manufactured by Merck Millipore) and subjected to HPLC.

For the HPLC, Ultimate 3000 (manufactured by Thermo Inc.), Corona charged aerosol detector (manufactured by Thermo Inc.) and TSKgel G6000PW (manufactured by Tosoh Corporation) were used. As the external standard product, P-82 pullulan standard (manufactured by Shodex) was used. The mobile phase used was distilled water in which 0.1% ammonium acetate was dissolved, and measurement was made by isocratic method for 110 minutes. The flow rate was 0.4 mL/min, and the column oven temperature was set at 40°C.

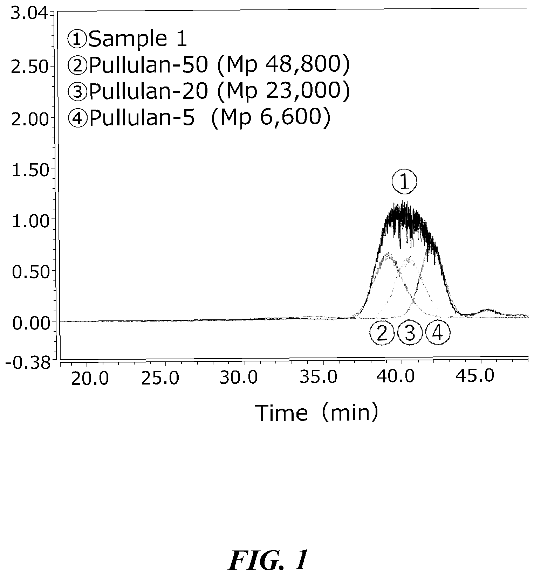

The HPLC charts of sample 1 and the external standard product are shown in Fig. 1. The Mp (peak top molecular weight) of P-5 is 6600. The Mp of P-20 is 23000, and the Mp of P-50 is 48800. Accordingly, the Mp of sample 1 under the measurement conditions was estimated to be less than 40000.

### <Test Example 1> Monoculture 1 of Faecalibacterium prausnitzii

### (1) Preparation of Media

Powder having the YCFA medium composition excluding the saccharides was added to the aqueous sodium alginate solution (sample 1) obtained in the Production Example described above, and a sodium alginate-containing YCFA medium was thus obtained.

For comparison, media containing a commercial sodium alginate material or a guar gum hydrolysate were prepared. It has already been known that the guar gum hydrolysate has the capability of growing *Faecalibacterium prausnitzii.* First, 0.3 mL of acetic acid (Kokusan Chemical Co., Ltd.) was added to 99.7 mL of MilliQ water, and the mixture was autoclaved at 104°C for 7.5 hours. The obtained sterilized water was subjected to SpeedVac to remove all the acetic acid and 70% of the water content, and an aqueous solution was thus obtained. A commercial sodium alginate material (sample 2: "ULV-L3 (molecular weight of 40000 to 60000)" (manufactured by KIMICA Corporation)) or a commercial guar gum hydrolysate (sample 3: "Sunfiber" (manufactured by Taiyo Kagaku Corporation)) in an amount of 1 g was added to the aqueous solution, and powder having the YCFA medium composition excluding the saccharides was further added. YCFA media were thus obtained. As a negative control, a medium without the samples to be tested was also prepared.

### (2) Monoculture

A vitamin solution and a cysteine solution which were sterilized by filtration were aseptically added to the YCFA media prepared in (1), and culture solutions having a final concentration of the sample to be tested of 1% (w/v) were thus prepared. The culture solutions were dispensed to a 96-well plate 353072 (manufactured by Falcon) each in a volume of 200 µL. Then, the 96-well plate was left to stand still in a Concept Plus (manufactured by Central Scientific Commerce, Inc.,) overnight, and the culture solutions were brought to anaerobic state. *Faecalibacterium prausnitzii* (MCC2041, deposited as an international deposit on September 20, 2019 to NITE Patent Microorganisms Depositary, National Institute of Technology and Evaluation (Room 122, 2-5-8 Kazusakamatari, Kisarazu-shi, Chiba 292-0818) and given the accession number of NITE BP-03027) was seeded thereto 24 hours before subjecting to the culture experiment and anaerobically cultured at 37°C, and the preculture solutions each in a volume of 6 µL were seeded to the culture solutions and anaerobically cultured for 48 hours at 37°C.

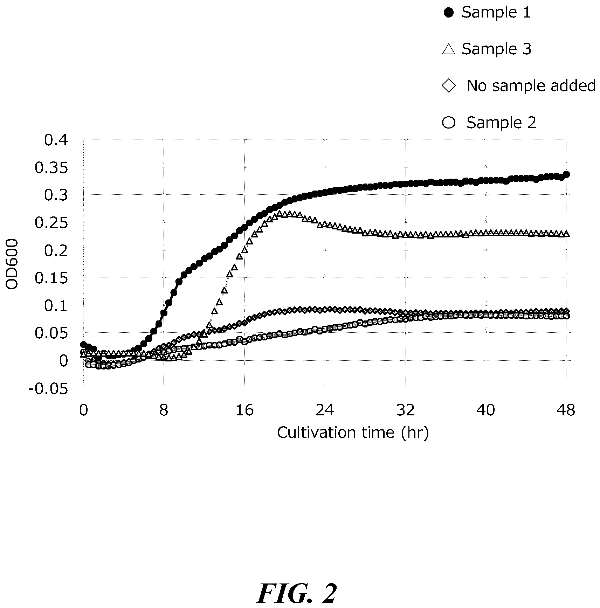

In Fig. 2, the changes in turbidities (OD₆₀₀) of the media with time are shown. Although *Faecalibacterium prausnitzii* could not assimilate the sodium alginate having a high molecular weight (sample 2) and grew slowly, *Faecalibacterium prausnitzii* assimilated the sodium alginate having a molecular weight reduced by hydrolysis (sample 1), and remarkable growth was observed. Moreover, sample 1 exhibited higher capability of growing *Faecalibacterium prausnitzii* than the guar gum hydrolysate (sample 3).

### <Test Example 2> Monoculture 2 of Faecalibacterium prausnitzii

Using a YCFA medium containing sample 1 prepared in the same manner as in Test Example 1, monoculture of *Faecalibacterium prausnitzii* (MCC2041, acceptance number: NITE ABP-03027) was conducted. The medium was taken 0 hour and 48 hours after starting the cultivation and subjected to HPLC.

For the HPLC, Ultimate 3000 (manufactured by Thermo Inc.), Corona charged aerosol detector (manufactured by Thermo Inc.) and TSKgel G3000PW (manufactured by Tosoh Corporation) were used. As the external standard product, P-82 pullulan standard (manufactured by Shodex) was used. The mobile phase used was distilled water in which 0.1% ammonium acetate was dissolved, and measurement was made by isocratic method for 110 minutes. The flow rate was 0.3 mL/min, and the column oven temperature was set at 40°C.

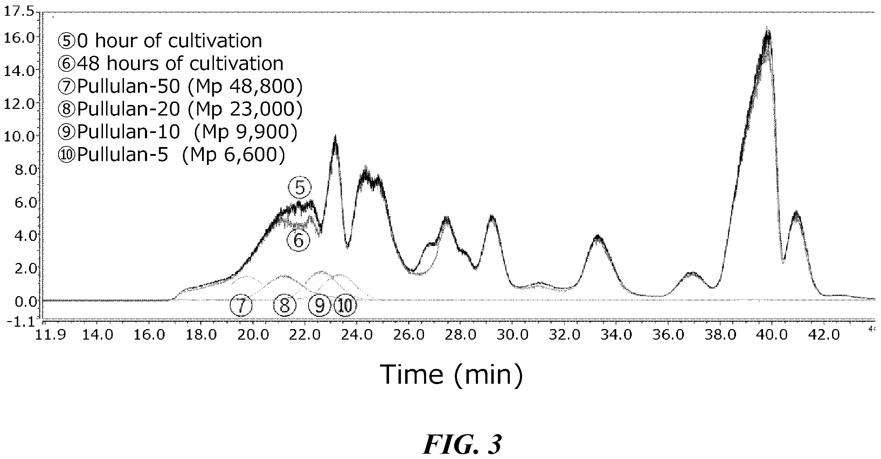

In Fig. 3, the charts of the medium obtained 0 hour and 48 hours after starting cultivation and the standards are shown together. Because the value in the range of the molecular weight of around 25000 to 10000 decreased throughout the cultivation and because the peak at around 20000 especially decreased, it is determined that alginic acid having a molecular weight in the range was assimilated by *Faecalibacterium prausnitzii.* In this regard, the decrease in the chart at around 27 minutes is considered to correspond to the consumption of the medium-derived components.

### <Test Example 3> Monoculture 3 of Faecalibacterium prausnitzii

Using a YCFA medium containing sample 1 prepared in the same manner as in Test Example 1, monoculture of *Faecalibacterium prausnitzii* (MCC2041, acceptance number: NITE ABP-03027) was conducted. The medium was taken 0 hour and 48 hours after starting the cultivation and subjected to HPLC.

For the HPLC, Ultimate 3000 (manufactured by Thermo Inc.), Refracto Max 521 (manufactured by Thermo Inc.) and TSKgel G3000PW (manufactured by Tosoh Corporation) were used. As the external standard product, P-82 pullulan standard (manufactured by Shodex) was used. The mobile phase used was distilled water in which 0.1M sodium nitrate was dissolved, and measurement was made by isocratic method for 80 minutes. The flow rate was 0.3 mL/min, and the column oven temperature was set at 40°C.

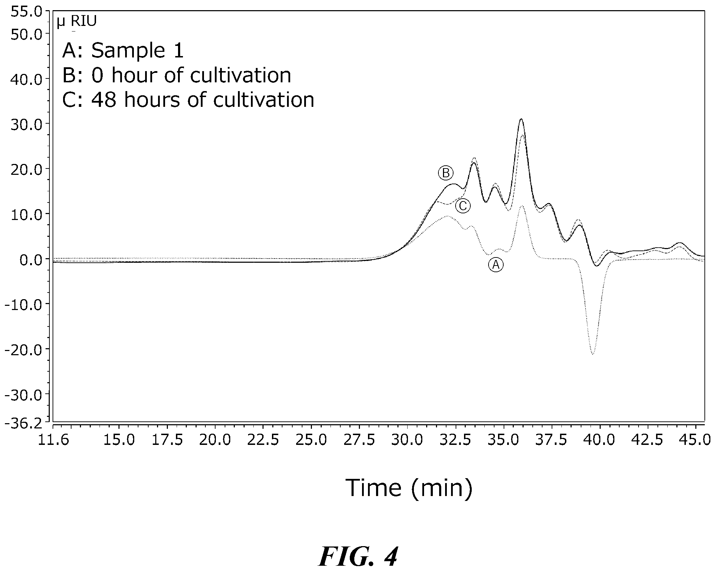

In Fig. 4, the charts of the medium obtained 0 hour and 48 hours after starting cultivation and sample 1 are shown together. Because the peak at around 31 to 32.5 minutes after starting the measurement decreased throughout the cultivation and because the peak is identical to the peak which appeared in sample 1, it can be seen that alginic acid having a molecular weight in the range was assimilated by *Faecalibacterium prausnitzii.*

The average molecular weight of the fraction corresponding to the peak decreased through the cultivation in Fig. 4, namely the alginic acid assimilated by *Faecalibacterium prausnitzii,* was calculated based on a calibration curve created from the elution times of the external standard product and was estimated to be about 3500. The average molecular weight of sample 1 was also calculated and was estimated to be about 6000.

### <Test Example 4> Analysis of Fraction Assimilated by Faecalibacterium prausnitzii

The size of alginic acid assimilated by *Faecalibacterium prausnitzii* was analyzed in more detail by thin-layer chromatography (TLC).

The culture solution obtained after 48 hours of cultivation in Test Example 3 and a YCFA medium containing sample 1 prepared in the same manner as in Test Example 1 were each centrifuged at 13000 × g at 4°C for three minutes, and the supernatants were collected and used as samples. As specimens, sample 1, commercial long-chain sodium alginate ("ULV-L3 (molecular weight of 40000 to 60000)" (manufactured by KIMICA Corporation)), commercial alginate oligosaccharides (mainly comprising di- to hexasaccharides, "alginate oligosaccharides (AOS)" (manufactured by Hokkaido Mitsui Chemicals, Inc.)), glucose, maltose and raffinose were prepared.

Each sample in a volume of 1 µL was spotted on an aluminum plate silica gel 60 for thin-layer chromatography (manufactured by Merck) and developed using a development solvent (formic acid:1-butanol:distilled water = 6:4:1). After spraying a diphenylamine·aniline·phosphoric acid reagent (100 mL acetone, 1 g diphenylamine, 1 mL aniline, 10 mL phosphoric acid), the saccharides were colored by heating.

Spots were observed from sample 1, which is a hydrolysate of long-chain sodium alginate. Because the spots were almost identical to the spots observed from alginate oligosaccharides, it was speculated that in sample 1, the long-chain sodium alginate was decomposed to oligosaccharides of di- to hexasaccharides. The spots were not observed from the long-chain sodium alginate.

Moreover, it was found that, in the culture solution, the spots corresponding to the oligosaccharides in sample 1, in particular the tetrasaccharides and the pentasaccharides, were thinner than those before the cultivation. This shows that *Faecalibacterium prausnitzii* assimilated saccharides having a some degree of polymerization, in particular the tetrasaccharides and the pentasaccharides, of the hydrolysate of the long-chain alginic acid.

## Claims

1. A prebiotic composition for a butyrate-producing bacterium comprising alginic acid and/or a salt thereof having a weight average molecular weight of 10000 or less.

2. The composition according to claim 1, wherein the butyrate-producing bacterium is a bacterium of *Faecalibacterium.*

3. The composition according to claim 1 or 2 which comprises the alginic acid and/or the salt thereof in an amount of 0.1 mass% or more of the entire composition.

4. The composition according to any one of claims 1 to 3 which is a food or a drink.

5. The composition according to any one of claims 1 to 3 which is a pharmaceutical product.

6. A method for producing a prebiotic composition for a butyrate-producing bacterium including a step of hydrolyzing long-chain alginic acid and/or a salt thereof to reduce the weight average molecular weight to 10000 or less.

7. The method according to claim 6, wherein the butyrate-producing bacterium is a bacterium of *Faecalibacterium.*

8. A method for producing a prebiotic food or drink for a butyrate-producing bacterium including
a step of hydrolyzing long-chain alginic acid and/or a salt thereof to reduce the weight average molecular weight to 10000 or less and
a step of adding the alginic acid and/or the salt thereof having a weight average molecular weight of 10000 or less obtained in the step to a food or drink material.

9. The method according to claim 8, wherein the butyrate-producing bacterium is a bacterium of *Faecalibacterium.*
